(19) Europäisches Patentamt
European Patent Office
Office européen des brevets

(11) Numéro de publication: **0 489 660 B1**

(12) # FASCICULE DE BREVET EUROPEEN

(45) Date de publication de fascicule du brevet: **02.11.94**

(51) Int. Cl.$^5$: **A01N 43/74**, C07D 277/30, C07D 263/32

(21) Numéro de dépôt: **91403293.3**

(22) Date de dépôt: **05.12.91**

(54) **Utilisation de thiazolylalkoxy acrylates pour la fabrication de compositions insecticides et/ou acaricides.**

(30) Priorité: **06.12.90 FR 9015289**

(43) Date de publication de la demande:
**10.06.92 Bulletin 92/24**

(45) Mention de la délivrance du brevet:
**02.11.94 Bulletin 94/44**

(84) Etats contractants désignés:
**CH DE FR GB IT LI NL**

(56) Documents cités:
**EP-A- 0 178 826**
**EP-A- 0 256 667**
**EP-A- 0 299 694**
**EP-A- 0 402 246**

(73) Titulaire: **ROUSSEL-UCLAF**
**35, Boulevard des Invalides**
**F-75007 Paris (FR)**

(72) Inventeur: **Benoit, Marc**
**Le Cannet Est - Pont de l'Etoile**
**F-13360 Roquevaire (FR)**
Inventeur: **Brayer, Jean-Louis**
**42, rue Jules Dubrulle**
**F-60440 Nanteuil le Haudoin (FR)**
Inventeur: **Demoute, Jean-Pierre**
**65, Avenue Foch**
**F-93360 Neuilly Plaisance (FR)**

(74) Mandataire: **Tonnellier, Marie-José et al**
**111, route de Noisy**
**B.P. no 9**
**F-93230 Romainville (FR)**

EP 0 489 660 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'utilisation de thiazolylalkoxyacrylates pour la fabrication de compositions insecticides et/ou acaricides.

La présente demande de brevet a pour objet l'utilisation des composés de formule (I) :

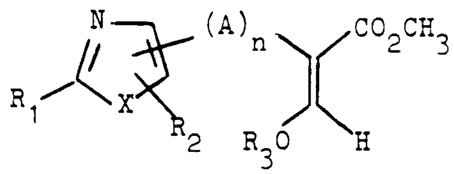

(I)

dans lesquels

- $R_1$ et $R_2$ identiques ou différents représentent un atome d'hydrogène, un atome d'halogène, un radical $CF_3$, un radical hydroxyle, un radical alkyle, alkényle, alkynyle ou thioalkyle renfermant jusqu'à 8 atomes de carbone éventuellement substitué, un radical alkoxy, thioalkoxy ou $SO_2$alkyle renfermant jusqu'à 8 atomes de carbone éventuellement substitué, un radical aryle, aryloxy ou thioaryle renfermant jusqu'à 18 atomes de carbone éventuellement substitué, un radical hétéroaryle ou hétéroaryloxy éventuellement substitué, un radical hétérocyclique à 5 ou 6 chaînons éventuellement substitué ou un radical

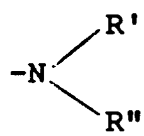

dans lequel les radicaux R′ et R″, identiques ou différents représentent un atome d'hydrogène ou un radical alkyle renfermant jusqu'à 8 atomes de carbone ou un radical aryle renfermant jusqu'à 18 atomes de carbone, éventuellement substitué,
- $R_3$ représente un radical alkyle, alkényle ou alkynyle renfermant jusqu'à 8 atomes de carbone éventuellement substitué ou un radical aryle, hétéroaryle renfermant jusqu'à 18 atomes de carbone, un radical hétérocyclique à 5 ou 6 chaînons ou un radical alkoxyalkoxyalkyle renfermant jusqu'à 14 atomes de carbone,
- X représente un atome d'oxygène ou de soufre ou un radical $NR_4$ dans lequel $R_4$ représente un atome d'hydrogène ou un radical alkyle, alkényle ou alkynyle, $SO_2$alkyle, $SO_2$alkényle ou $SO_2$alkynyle renfermant jusqu'à 8 atomes de carbone ou un radical aryle ou $SO_2$aryle renfermant jusqu'à 18 atomes de carbone éventuellement substitué,
- n représente le nombre 0 ou le nombre 1,
- A représente un radical choisi dans le groupe des radicaux suivants :

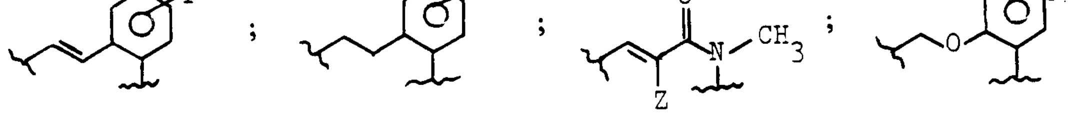

Y représentant un atome d'hydrogène, un atome d'halogène, un radical $CF_3$, un radical alkyle ou alkoxy renfermant juqu'à 8 atomes de carbone et Z représentant un atome d'hydrogène ou un atome d'halogène,
- la géométrie des doubles liaisons étant E ou Z ou un mélange de géométrie E et Z (au sens de Cahn, Ingold, Prelog), pour la fabrication de compositions insecticides et/ou acaricides.

Dans la définition des différents substituants,
- alkyle représente de préférence un radical méthyle, éthyle, propyle, isopropyle, butyle, isobutyle, sec-butyle ou tert-butyle,

- alkényle représente de préférence un radical vinyle, allyle ou 1,1-diméthylallyle,
- alkynyle représente de préférence un radical éthynyle ou propynyle,
- alkoxy représente de préférence un radical méthoxy, éthoxy, propoxy, isopropoxy, butoxy linéaire ou ramifié, pentoxy linéaire ou ramifié,
- aryle représente de préférence un radical phényle,
- aryloxy représente de préférence un radical phényloxy,
- hétéroaryle représente de préférence un radical furyle, pyridyle, pipérazinyle, benzofurannyle, isobenzofurannyle, oxazolyle ou isoxazolyle, thiazolyle, thiophényle,
- hétérocyclique représente de préférence un radical morpholinyle, pyrannyle, pyridazinyle, dioxolyle,
- alkoxyalkoxyalkyle représente de préférence un radical méthoxyéthoxyalkyle ou tout autre radical de formule :

  $(CH_2)_p$-O-$(CH_2)_{p'}$-O-alkyle

  p et p' identiques ou différents représentant les nombres 1, 2, 3 ou 4 et "alkyle" représentant un radical alkyle renfermant jusqu'à 8 atomes de carbone,
- halogène représente de préférence un atome de fluor ou de chlore.

Lorsque les différents radicaux ci-dessus sont substitués, ils portent de préférence des substituants choisis dans le groupe constitué par les radicaux hydroxyles libres, estérifiés ou éthérifiés dans lesquels la partie ester ou éther renferme de 1 à 18 atomes de carbone, comme par exemple le radical acétoxy ou le radical méthoxy, les fonctions cétones et oximes, les radicaux alkyles linéaires, ramifiés ou cyclisés, saturés ou insaturés, comportant jusqu'à 18 atomes de carbone et éventuellement interrompus par des hétéroatomes, par exemple le radical méthyle, éthyle, propyle, isopropyle ou méthylènedioxy, le radical éthényle $-CH=CH_2$ ou le radical éthynyle -C≡CH, les atomes d'halogène, les groupements $CF_3$, $SCF_3$, $OCF_3$, $NO_2$, $NH_2$ ou C≡N.

Dans la définition des radicaux thioalkyle, thioalkoxy, thioaryle, $SO_2$alkyle, $SO_2$alkényle, $SO_2$alkynyle, $SO_2$aryle, hétéroaryloxy, alkoxyalkoxyalkyle, il va de soi que les substituants alkyle, alkoxy, alkényle, alkynyle, aryle et aryloxy sont choisis de préférence parmi les radicaux cités ci-dessus.

L'invention a particulièrement pour objet l'utilisation des composés de formule (I') :

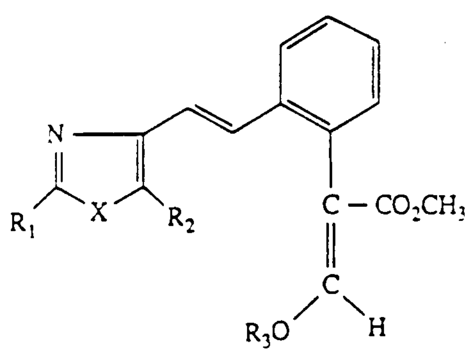

(I')

dans laquelle $R_1$, $R_2$, $R_3$ et X ont la signification indiquée ci-dessus, la géométrie des doubles liaisons étant E ou Z ou un mélange de géométrie E et Z, pour la fabrication de compositions insecticides et/ou acaricides.

L'invention a particulièrement pour objet l'utilisation caractérisée en ce que la géométrie de la double liaison entre l'hétérocycle et le noyau aromatique est (E) et l'utilisation caractérisée en ce que la géométrie de la double liaison du groupement alkoxyacrylate est (E).

L'invention a plus particulièrement pour objet l'utilisation des composés de formule (I) dans lesquels $R_2$ représente un atome d'hydrogène.

L'invention a tout particulièrement pour objet l'utilisation caractérisée en ce que X représente un atome de soufre ou d'oxygène, l'utilisation caractérisée en ce que $R_1$ représente un radical alkyle, renfermant jusqu'à 3 atomes de carbone éventuellement substitué, par exemple un radical éthyle, isopropyle ou trifluorométhyle, l'utilisation caractérisée en ce que $R_1$ représente un radical phényle non substitué ou substitué par un ou plusieurs atomes d'halogène, par exemple un ou plusieurs atomes de chlore, de brome ou de fluor, par un ou plusieurs radicaux trifluorométhyle, trifluorométhoxy ou par un radical méthylènedioxy, ainsi que l'utilisation caractérisée en ce que $R_1$ représente un radical benzyle.

Parmi les utilisations préférées, on peut citer celles dans lesquelles le composé de formule (I) est l'un des composés suivants :

- alpha-[(E)-(méthoxyméthylène)] 2-[(E)-2-[2-(2-éthyl 4-thiazolyl) 4-thiazolyl] éthényl] benzèneacétate de méthyle,
- alpha-[(E)-(méthoxyméthylène)] 2-[(E)-2-[2-(3,4-dichloro phényl) 4-thiazolyl] éthényl] benzèneacétate de méthyle,
- alpha-[(E)-(méthoxyméthylène)] 2-[(E)-2-(2-trifluorométhyl) 4-thiazolyl) éthényl] benzèneacétate de méthyle,
- alpha-[(E)-(méthoxyméthylène)] 2-[(E)-2-[2-(4-chlorophényl) 4-thiazolyl) éthényl] benzèneacétate de méthyle,
- alpha-[(E)-(méthoxyméthylène)] 2-[(E)-2-[2-(2-fluorophényl) 4-thiazolyl) éthényl] benzèneacétate de méthyle,
- alpha-[(E)-(méthoxyméthylène)] 2-[(E)-2-[2-(3-trifluorométhyl4-bromophényl) 4-thiazolyléthényl] benzèneacétate de méthyle,
- alpha-[(E)-(méthoxyméthylène)] 2-[(E)-2-[2-(1,3-benzodioxol-5-yl) 4-thiazolyl) éthényl] benzèneacétate de méthyle,
- alpha-[(E)-(méthoxyméthylène)] 2-[(E)-2-[2-(4-trifluorométhylphényl) thiazolyl) éthényl] benzèneacétate de méthyle,
- alpha-[(E)-(méthoxyméthylène)] 2-[(E)-2-[2-(4-trifluorométhoxyphényl) 4-thiazolyl) éthényl] benzèneacétate de méthyle,
- alpha-[(E)-(méthoxyméthylène)] 2-[(E)-2-[2-(3,4-dichloro phényl) 4-oxazolyl] éthényl] benzèneacétate de méthyle.

La plupart des produits de formule (I) sont des produits connus. Ils sont décrits et revendiqués dans la demande de brevet européen EP.A. 0402246.

Dans cette demande de brevet, les produits de formule (I) sont présentés comme doués de propriétés fongicides.

On vient de découvrir que les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme DIABROTICA, les taupins, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens parasites des végétaux.

Les composés de formule (I) peuvent encore être utilisés pour lutter contre les acariens parasites des animaux, pour lutter par exemple contre les tiques et notamment les tiques du genre de Boophilus, celles du genre Hyalomnia, celles du genre Amblyomnia et celles du genre Rhipicéphalus ou pour lutter contre toutes sortes de gales et notamment la gale sarcoptique, la gale psoroptique et la gale chorioptique.

Ces compositions insecticides et/ou acaricides sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement être utilisées, pour la préparation de serpentin insecticide (ou coil) combustible, ou encore constituées d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les compositions insecticides selon l'invention, comme les compositions acaricides peuvent être additionnées éventuellement d'un ou plusieurs autres agents pesticides. Les compositions acaricides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Les composés acaricides selon l'invention sont utilisés, de préférence, à des doses comprises entre 1 et 100 g de matière active à l'hectare.

Les composés de formule (I) présentent une excellente tolérance générale, et l'invention a donc également pour objet les produits de formule (I), pour lutter notamment contre les affections créées par les tiques et les gales chez l'homme et l'animal.

Les produits de formule (I) sont notamment utilisés pour lutter contre les poux à titre préventif ou curatif et pour lutter contre la gale.

Les produits de formule (I) peuvent être administrés par voie externe, par vaporisation, par shampooing, par bain ou badigeonnage.

Les produits de formule (I) à usage vétérinaire peuvent être également administrés par badigeonnage de l'épine dorsale selon la méthode dite méthode "pour-on".

Certains composés de formule (I), inclus dans la formule générale de la demande de brevet européen n° EP.A. 0402246 citée ci-dessus ne sont pas décrits dans cette demande de brevet et sont en eux-mêmes un objet de la présente invention : il s'agit notamment des composés répondant à la formule (I') telle que définie ci-dessus dont les noms suivent :

- alpha-[(E)-(méthoxyméthylène)] 2-[(Z)-2-[2-(4,6-dichlorophényl) 4-thiazolyl] éthényl] benzèneacétate de méthyle,
- alpha-[(E)-(méthoxyméthylène)] 2-[(E)-[2-(2-trifluorométhyl) 4-thiazolyl] éthényl] benzèneacétate de méthyle.
- alpha-[(E)-(méthoxyméthylène)] 2-[(E)-2-[2-(2-éthyl 4-thiazolyl) 4-thiazolyl] éthényl] benzèneacétate de méthyle,
- alpha-[(E)-(méthoxyméthylène)] 2-[(E)-2-[2-(3,4-dichloro phényl) 4-thiazolyl] éthényl] benzèneacetate de méthyle,
- alpha-[(E)-(méthoxyméthylène)] 2-[(E)-2-(2-trifluorométhyl) 4-thiazolyl) éthényl] benzèneacétate de méthyle,
- alpha-[(E)-(méthoxyméthylène)] 2-[(E)-2-[2-(4-chlorophényl) 4-thiazolyl) éthényl] benzèneacétate de méthyle,

- alpha-[(E)-(méthoxyméthylène)] 2-[(E)-2-[2-(2-fluorophényl) 4-thiazolyl) éthényl] benzèneacétate de méthyle,
- alpha-[(E)-(méthoxyméthylène)] 2-[(E)-2-[2-(3-trifluorométhyl4-bromophényl) 4-thiazolyléthényl] benzèneacétate de méthyle,
- alpha-[(E)-(méthoxyméthylène)] 2-[(E)-2-[2-(1,3-benzodioxol-5-yl) 4-thiazolyl) éthényl] benzèneacétate de méthyle,
- alpha-[(E)-(méthoxyméthylène)] 2-[(E)-2-[2-(4-trifluorométhylphényl) thiazolyl) éthényl] benzèneacétate de méthyle,
- alpha-[(E)-(méthoxyméthylène)] 2-[(E)-2-[2-(4-trifluorométhoxyphényl) 4-thiazolyl) éthényl] benzèneacétate de méthyle,
- alpha-[(E)-(méthoxyméthylène)] 2-[(E)-2-[2-(3,4-dichloro phényl) 4-oxazolyl] éthényl] benzèneacétate de méthyle.

Ces composés de formule (I) peuvent être préparés selon un mode opératoire indiqué dans la demande de brevet européen citée ci-dessus.

Ces composés de formule (I), comme tous les composés de formule (I') telle que définie précédemment et notamment les composés de formule (I' dans laquelle $R_1$ et $R_2$ identiques ou différents ne représentent pas un radical hydroxyle ou un radical

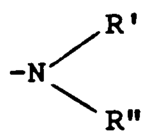

dans laquelle R' et R'' ont la signification indiquée précédemment, peuvent également être préparés comme indiqué dans la demande de brevet français 90-14496.

Selon ce procédé, l'on soumet un composé de formule :

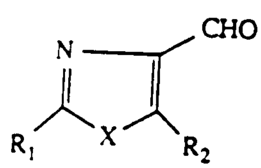

(A)

dans laquelle X, $R_1$ et $R_2$ conservent leur signification précédente, à l'action d'un halogénure de formule :

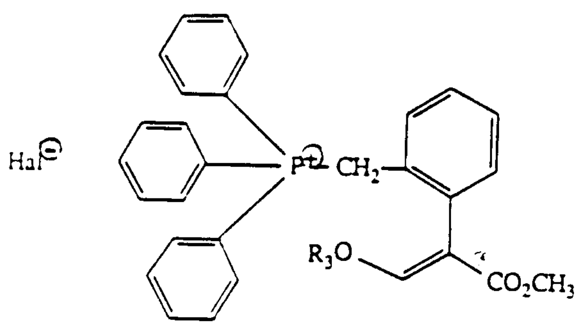

(B)

dans laquelle $R_3$ conserve sa signification précédente et Hal représente un atome d'halogène, en présence d'une base, pour obtenir le composé de formule (I') correspondant que l'on sépare si désiré en chacun des isomères.

Dans cette demande de brevet français, les composés de formule (B) sont décrits et revendiqués, ainsi que leur procédé de préparation selon le schéma suivant :

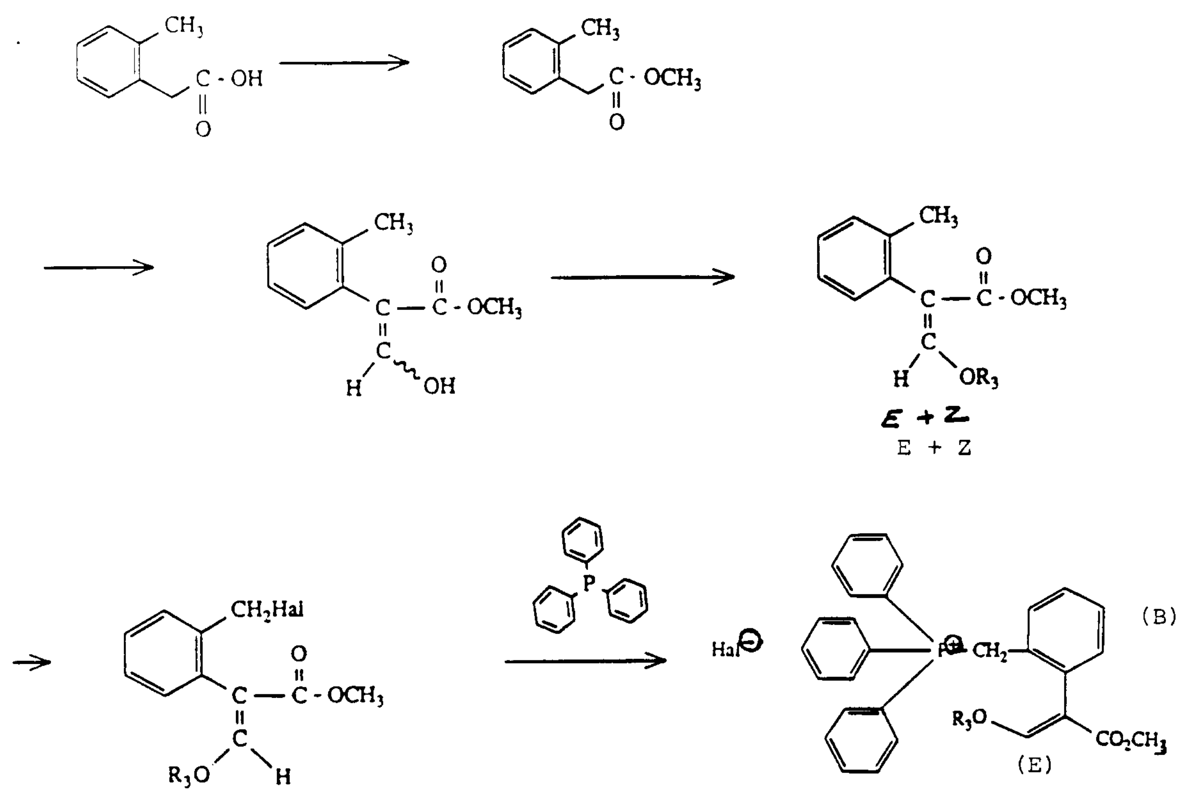


La base utilisée lors de la réaction des composés (A) et (B) pour préparer les produits de formule (I'), peut être par exemple l'hydrure de sodium, un alcoolate alcalin ou alcalino terreux, un amidure alcalin, une amine secondaire ou tertiaire en présence de bromure de lithium.

Dans la demande de brevet français n° 90 14496 citée précédemment, il est également décrit un procédé selon lequel l'inversion de la configuration des isomères au niveau de la double liaison comprise entre l'hétérocycle à 5 chaînons et le noyau aromatique est réalisée par action de l'iode.

Les composés de formule (I) et notamment les composés de formule (I') définis ci-dessus dans lequel $R_1$ et $R_2$ identiques ou différents, représentent un radical hydroxyle ou un radical

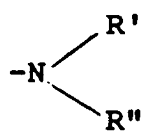

dans lequel R' et R" ont la signification précédente, sont des composés nouveaux. Ils peuvent être préparés comme indiqué dans les demandes de brevet citées précédemment.

L'invention a donc pour objet les composés de formule (I) dans laquelle $R_1$ et $R_2$ identiques ou différents représentent un radical hydroxyle ou un radical

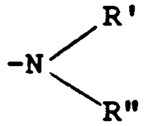 ,

R' et R" ayant la signification précédente et plus particulièrement les composés de formule (I') dans laquelle $R_1$ et $R_2$ identiques ou différents représentent un radical hydroxyle ou un radical

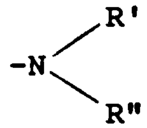 ,

R′ et R″ ayant la signification précédente.

Les produits suivants correspondants aux exemples 1 à 50 sont préparés comme indiqué dans la demande de brevet européen n° EP.A. 0402246.

Exemple 1 : (E,Z) 3-méthoxy 2-[méthyl [1-oxo 3-[2-(trifluoro-méthyl) 4-thiazolyl] 2-propényl] amino] 2-propènoate de méthyle.

Exemple 2 : (E,Z) 3-[[(2-méthoxy) éthoxy] méthoxy] 2-[méthyl-[1-oxo 3-[2-(trifluorométhyl) 4-thiazolyl] 2-propényl] amino]] 2-propénoate de méthyle.

Exemple 3 : 2-(2-méthylthio 4-thiazolyl) 3-méthoxy 2-propénoate de méthyle (isomère E) et (isomère Z).

Exemple 4 : 2-(2-méthylthio 4-thiazolyl) 3-(2-méthoxyéthoxy) méthyloxy propénoate de méthyle (isomère Z) et (isomère E).

Exemple 5 : (E,Z) 3-méthoxy [2-méthyl [1-oxo 3-(4-thiazolyl) 2-propényl] amino] 2-propénoate de méthyle.

Exemple 6 : (E,Z) 3-[[(2-méthoxy) éthoxy] méthoxy] 2-[méthyl [1-oxo 3-(4-thiazolyl) 2-propényl] amino] 2-propénoate de méthyle.

Exemple 7 : 2-(4-chlorophényl) alpha-(méthoxy méthylène) 4-thiazole acétate de méthyle isomères (E) + (Z).

Exemple 8 : 2-(4-chlorophényl) alpha-3-[[2-méthoxy (éthoxy)] méthoxy] 4-thiazole acétate de méthyle isomères (E) + (Z).

Exemple 9 : 4-(4-chlorophényl) 2-(4-cyclopropylphényl) alpha-(méthoxy méthylène 5-thiazole acétate de méthyle isomères E et Z.

Exemple 10 : 4-(4-chlorophényl) 2-(4-cyclopropylphényl) alpha-[[(2-méthoxy éthoxy) méthoxy] méthylène] 5-thiazole acétate de méthyle isomères E et Z.

Exemple 11 : 2-(2-méthylthio 4-méthyl 5-thiazolyl) 3-méthoxy 2-propénoate de méthyle (isomères E et Z).

Exemple 12 : 2-(2-méthylthio 4-méthyl 5-thiazolyl) 3-(2-méthoxyéthoxy) méthoxy propénoate de méthyle (isomères Z et E).

Exemple 13 : 2-[N-[3-[2'-éthyl (2,4'-bithiazol) 4-yl] 1-oxo 2-(E)-propényl] méthylamino] 3-(Z)-méthoxy propénoate de méthyle.

Exemple 14 : 2-[N-[3-[2'-éthyl (2,4'-bithiazol) 4-yl] 1-oxo 2-(Z)-fluoro propényl] méthylamino] 3-méthoxy (Z) propénoate de méthyle.

Exemple 15 : (Z,Z) 2-[(2-fluoro 1-oxo 3-(4-thiazolyl) 2-propényl) N-méthylamino] 3-méthoxy 2-propénoate de méthyle.

Exemple 16 : (E,E) alpha-(méthoxy méthylène) 2-[2-(4-thiazolyl) éthényl] benzène acétate de méthyle.

Exemple 17 : 2-(E)-[[[2'-éthyl (2,4'-bithiazol) 4-yl] éthényl] 2-phényl] 3-méthoxy 2-(E)-propénoate de méthyle.

EXEMPLE 18 : 2-(alpha)-(méthoxyméthylène) 2'-éthyl (2,4'-bithiazol) 4-acétate de méthyle.

EXEMPLE 19 : (Z,E) 3-méthoxy 2-[méthyl [2-fluoro 1-oxo 3-[2-(trifluorométhyl) 4-thiazolyl] 2-propényl] amino] 2-propènoate de méthyle.

EXEMPLE 20 : alpha-(méthoxyméthylène) 2-[(2-phényl 4-thiazolyl) méthoxy] benzène acétate de méthyle.

EXEMPLE 21 : alpha (E)-(méthoxyméthylène) 2-(E)-[[2-(2-pentyl) 4-thiazolyl] éthényl] benzène acétate de méthyle.

EXEMPLE 22 : alpha (Z)-(méthoxyméthylène) 2-(E)-[[2-(2-pentyl) 4-thiazolyl] éthényl] benzène acétate de méthyle.

EXEMPLE 23 : (E) alpha-(méthoxyméthylène) 2-[(2-pentyl) 4-thiazolyl] méthoxy] benzène acétate de méthyle.

EXEMPLE 24 : (Z) alpha-(méthoxyméthylène) 2-[(2-pentyl) 4-thiazolyl] méthoxy] benzène acétate de méthyle.

EXEMPLE 25 : alpha (E) (méthoxyméthylène) 2-[(2-(2-méthylpropen-1-yl) 4-thiazolyl] méthoxy] benzène acétate de méthyle.

EXEMPLE 26 : (Z) alpha-(méthoxyméthylène) 2-[(4-thiazolyl 2-méthyléthyl) méthoxy] benzène acétate de méthyle.

EXEMPLE 27 : (E) alpha-(méthoxyméthylène) 2-[(4-thiazolyl 2-méthyléthyl) méthoxy] benzène acétate de méthyle.
EXEMPLE 28 : (E) 2-[[2'-éthyl (2,4'-bithiazol)-4-yl] méthoxy] alpha-(méthoxyméthylèn) benzène acétate de méthyle.
EXEMPLE 29 : alpha (E)-(méthoxyméthylène) 2-(E)-[[2-(2-pentyl) 4-thiazolyl] éthényl] benzène acétate de méthyle.
EXEMPLE 30 : (E)-alpha-(méthoxyméthylène) 2-[(4-thiazolyl 2-méthyléthyl] éthoxy] benzène acétate de méthyle.
EXEMPLE 31 : E,E alpha-(méthoxyméthylène) 2-[2-(2-méthyléthyl 4-thiazolyl) éthényl] benzène acétate de méthyle.
EXEMPLE 32 : E,Z alpha-(méthoxyméthylène) 2-[2-(2-méthyléthyl 4-thiazolyl) éthényl] benzène acétate de méthyle.
EXEMPLE 33 : alpha (Z)-(méthoxyméthylène) 2-(E)-[[2-(2-pentyl) 4-thiazolyl] éthényl] benzène acétate de méthyle.
EXEMPLE 34 : alpha (E)-(méthoxyméthylène) 2-(E)-[(2-méthyl 4-thiazolyl) éthényl] benzène acétate de méthyle.
EXEMPLE 35 : alpha (Z)-(méthoxyméthylène) 2-(E)-[(2-méthyl 4-thiazolyl) éthényl] benzène acétate de méthyle.
EXEMPLE 36 : alpha (E)-(méthoxyméthylène) 2-(E)-[[2-(2-méthyl 1-propényl) 4-thiazolyl] éthényl] benzène acétate de méthyle.
EXEMPLE 37 : alpha (Z)-(méthoxyméthylène) 2-(E)-[[2-(2-méthyl 1-propényl) 4-thiazolyl] éthényl] benzène acétate de méthyle.
EXEMPLE 38 : alpha-(méthoxyméthylène) 2-[(2-méthyl 4-thiazolyl) méthoxy] benzène acétate de méthyle.
EXEMPLE 39 : alpha-(méthoxyméthylène) 2-[(2-chloro 4-thiazolyl) méthoxy] benzène acétate de méthyle.
EXEMPLE 40 : alpha-(méthoxyméthylène) 2-[(2-méthoxy 4-thiazolyl) méthoxy] benzène acétate de méthyle.
EXEMPLE 41 : alpha-(méthoxyméthylène) 2-[(2-méthylthio 4-thiazolyl) méthoxy] benzène acétate de méthyle.
EXEMPLE 42 : alpha (E)-(méthoxyméthylène) 2-(E)-[[2-(2-méthylthio 4-thiazolyl) éthényl] benzène acétate de méthyle.
EXEMPLE 43 : alpha (E)-(méthoxyméthylène) 2-(E)-[[2-(2-chloro 4-thiazolyl) éthényl] benzène acétate de méthyle.
EXEMPLE 44 : (E) alpha-(méthoxyméthylène) 2-[[4-oxazolylméthyl] méthoxy] benzène acétate de méthyle.
EXEMPLE 45 : E,E alpha-(méthoxyméthylène) 2-[2-(2-méthyl 4-oxazolyl) éthényl] benzène acétate de méthyle.
EXEMPLE 46 : alpha (E)-(méthoxyméthylène) 2-(E)-[[2-(2-méthoxy 4-thiazolyl) éthényl] benzène acétate de méthyle.
EXEMPLE 47 : (E,E) alpha-(méthoxyméthylène) 2-[2-(4-thiazolyl) éthényl] benzène acétate de méthyle
EXEMPLE 48 : 2-(E) [[[2'-éthyl (2,4'-bithiazol) 4-yl] éthényl] 2-phényl] 3-méthoxy 2-(E) propénoate de méthyle
EXEMPLE 49 : alpha (E) (méthoxyméthylène) 2-(E)-[[2-(2-pentyl) 4-thiazolyl] éthényl] benzène acétate de méthyle
EXEMPLE 50 : Z,E alpha-(méthoxy méthylène) 2-[2-[2-(2,4-dichlorophényl) 4-thiazolyl] éthényl] benzèneacétate de méthyle

En utilisant les procédés de préparation indiqués dans la demande de brevet européen citée ci-dessus, procédé selon lequel on soumet un composé de formule (D) :

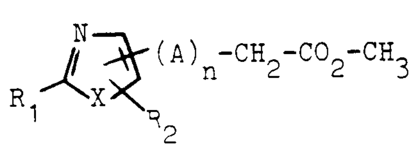

**(D)**

dans laquelle X, $R_1$, $R_2$, A et n conservent leur signification précédente, à l'action d'une base forte et d'un formiate d'alcoyle ou du N-formyl- imidazole pour obtenir le composé de formule (E) :

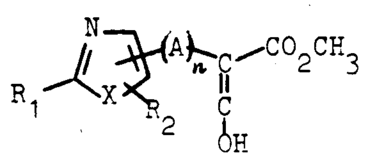

(E)

que l'on soumet à l'action d'un agent capable d'introduire le radical $R_3$ pour obtenir le composé de formule (I) correspondant et sépare le cas échéant les différents isomères, ou bien, procédé selon lequel on soumet suivant la réaction de Wittig-Horner un composé de formule (F) :

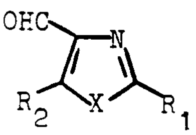

(F)

dans laquelle X, $R_1$ et $R_2$ conservent leurs significations précédentes à l'action d'un composé de formule (G) :

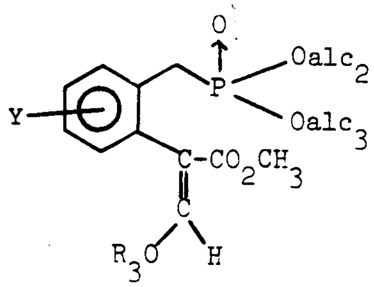

(G)

dans laquelle Y et $R_3$ conservent leurs significations précédentes, $alc_2$ et $alc_3$ identiques ou différents réprésentent un radical alkyle renfermant jusqu'à 8 atomes de carbone, pour obtenir le composé de formule (I') correspondant

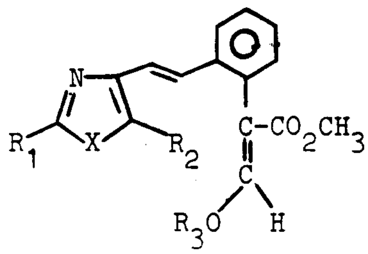

(I')

dans laquelle $R_1$, $R_2$ et $R_3$ ont la signification précédente, on a préparé les produits des exemples 51 à 169.

Les tableaux ci-dessous résument les principales caractéristiques des produits obtenus dans les exemples ; les points de fusion lorsque les produits concernés ont un point de fusion.

TABLEAU I

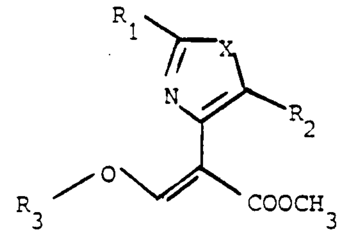

| EX | X | R1 | R2 | R3 | isomère | F°C | δH |
|---|---|---|---|---|---|---|---|
| 8 | S | 4ClC6H4 | H | CH2-O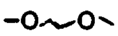 | Z | - | 8,13 |
| 4 | S | S-CH3 | H | CH2-O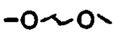 | Z | - | 7,98 |
| 7 | S | 4-ClC6H4 | H | CH3 | Z | 109 | 7,9 |
| 3 | S | S-CH3 | H | CH3 | Z | 50 | 7,84 |
| 3 | S | S-CH3 | H | CH3 | E | - | 7,59 |
| 7 | S | 4-ClC6H4 | H | CH3 | E | - | 7,64 |
| 8 | S | 4-ClC6H4 | H | CH2-O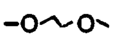 | E | 50 | 7,85 |
| 4 | S | S-CH3 | H | CH2-O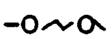 | E | - | 7,79 |
| 18 | S | i | H | CH3 | E | 72 | - |

i = 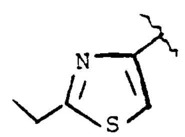

δH : déplacement chimique en ppm du proton de la fonction bêta méthoxy acrylate par rapport à une référence interne-tétraméthylsilane

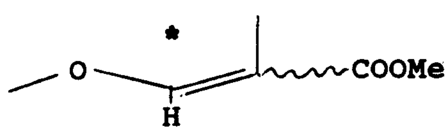

TABLEAU II

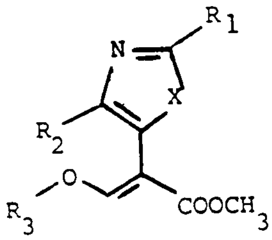

| EX | X | R1 | R2 | R3 | isomère | F°C | δH |
|---|---|---|---|---|---|---|---|
| 9 | S | 4-CyprC6H4 | 4-ClC6H4 | CH3 | E | 153 | 6,73 |
| 10 | S | 4-CyprC6H4 | 4-ClC6H4 | 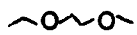 | E | 82 | 6,97 |
| 9 | S | 4-CyprC6H4 | 4-ClC6H4 | CH3 | Z | 114 | 7,53 |
| 10 | S | 4-CyprC6H4 | 4-ClC6H4 | 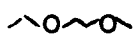 | Z | - | 7,77 |
| 11 | S | SCH3 | CH3 | CH3 | Z | - | 7,6 |
| 11 | S | SCH3 | CH3 | CH3 | E | - | 6,72 |
| 12 | S | SCH3 | CH3 | 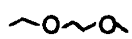 | Z | - | 7,84 |
| 12 | S | SCH3 | CH3 | 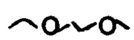 | E | - | 6,96 |

## TABLEAU III

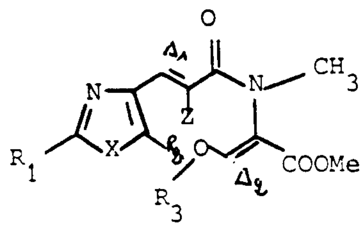

| EX | X | R1 | R2 | Z | R3 | 1 | 2 | F°C | Δ 2 |
|---|---|---|---|---|---|---|---|---|---|
| 9 | S | CF3 | H | H | CH3 | E | Z | 104 | 7,49-7,52 |
| 14 | S | i | H | F | CH3 | Z | Z | 99 | 7,35 |
| 5 | S | H | H | F | CH3 | E | Z | 108 | 7,46 |
| 6 | S | H | H | H | 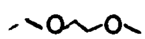 | E | Z | 89 | 7,72 |
| 13 | S | i | H | H | CH3 | E | Z | - | 7,36 |
| 15 | S | H | H | F | CH3 | Z | Z | - | 7,34 |
| 2 | S | CF3 | H | H | 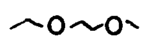 | E | Z | 76 | 7,77 |
| 19 | S | CF3 | H | F | CH3 | Z | E | - | - |

i = 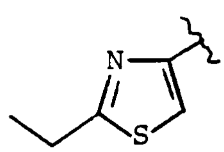

TABLEAU IV

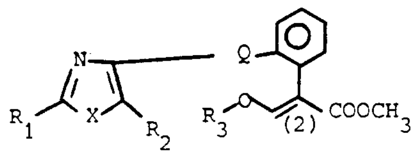

| EX | X | R1 | R2 | Q | R3 | Q | (2) | F°C |
|---|---|---|---|---|---|---|---|---|
| 17 | S | i | H | -CH=CH- | CH3 | E | E | 141 |
| 16 | S | H | H | -CH=CH- | CH3 | E | E | 106 |
| 20 | S | C6H5 | H | CH2-O | CH3 | - | E | 89 |
| 21 | S | C6H5 | H | -CH=CH- | CH3 | E | E | 115 |
| 22 | S | C6H5 | H | -CH=CH- | CH3 | E | Z | 131 |
| 23 | S | nC5H11 | H | CH2-O | CH3 | - | E | - |
| 24 | S | nC5H11 | H | CH2-O | CH3 | - | Z | - |
| 25 | S | CH=C(CH3)-CH3 | H | CH2-O | CH3 | - | E | - |
| 26 | S | iC3H7 | H | CH2-O | CH3 | - | Z | - |
| 27 | S | H | H | CH2-O | CH3 | - | E | 67 |
| 28 | S | i | H | CH2-O | CH3 | - | E | 112 |
| 29 | S | nC5H11 | H | -CH=CH- | CH3 | E | E | - |
| 30 | S | iC3H7 | H | CH2-O | CH3 | - | E | - |
| 31 | S | iC3H7 | H | -CH=CH- | CH3 | E | E | 92 |
| 32 | S | iC3H7 | H | -CH=CH- | CH3 | E | Z | - |
| 33 | S | nC5H11 | H | -CH=CH- | CH3 | E | Z | - |
| 34 | S | CH3 | H | -CH=CH- | CH3 | E | E | 115 |
| 35 | S | CH3 | H | -CH=CH- | CH3 | E | Z | 136 |
| 36 | S | -CH=C(CH3)-CH3 | H | -CH=CH- | CH3 | E | E | 103 |
| 37 | S | -CH=C(CH3)-CH3 | H | -CH=CH- | CH3 | E | Z | 134 |
| 38 | S | CH3 | H | CH2O | CH3 | - | E | - |
| 39 | S | Cl | H | CH2O | CH3 | - | E | 97 |
| 40 | S | OCH3 | H | CH2O | CH3 | - | E | 83 |
| 41 | S | SCH3 | H | CH2O | CH3 | - | E | 89 |
| 42 | S | SCH3 | H | -CH=CH- | CH3 | E | E | 124 |
| 43 | S | Cl | H | -CH=CH- | CH3 | E | E | 114 |
| 44 | O | CH3 | H | CH2O | CH3 | - | E | - |
| 45 | O | CH3 | H | -CH=CH- | CH3 | E | E | 88 |
| 46 | S | OCH3 | H | -CH=CH- | CH3 | E | E | 112 |
| 47 | S | H | H | -CH=CH- | CH3 | E | E | 106 |
| 48 | S | i | H | -CH=CH- | CH3 | E | E | 141 |
| 49 | S | nC5H11 | H | -CH=CH- | CH3 | E | E | - |
| 50 | S | 2,4Cl2C6H3 | H | -CH=CH- | CH3 | Z | E | - |
| 51 | S | 2,4Cl2C6H3 | H | -CH=CH- | CH3 | E | E | 170 |
| 52 | S | 3ClC6H4 | H | -CH=CH- | CH3 | E | E | 147 |
| 53 | S | 3.4Cl2C6H3 | H | CH2O | CH3 | - | E | 130 |
| 54 | S | 3.4Cl2C6H3 | H | CH2O | CH3 | - | Z | 139 |
| 55 | S | 3.4Cl2C6H3 | H | -CH=CH- | CH3 | E | E | 183 |
| 56 | S | 3.4Cl2C6H3 | H | -CH=CH- | CH3 | Z | Z | 137 |

i =

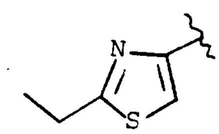

TABLEAU IV

(suite)

| EX | X | R1 | R2 | Q | R3 | Q | (2) | F°C |
|---|---|---|---|---|---|---|---|---|
| 57 | S | 4Cl3OCH3C6H3 | H | -CH=CH- | CH3 | E | E | 133 |
| 58 | S | CF3 | H | -CH=CH- | CH3 | E | E | 113 |
| 59 | S | CF3 | H | -CH=CH- | CH3 | Z | E | - |
| 60 | S | CF3 | H | CH2-O | CH3 | - | E | - |
| 61 | O | C6H5 | H | -CH=CH- | CH3 | E | E | 136 |
| 62 | O | C6H5 | H | -CH=CH- | CH3 | Z | E | - |
| 63 | O | C6H5 | H | CH2-O | CH3 | - | Z | 100 |
| 64 | S | CH2-C6H5 | H | -CH=CH- | CH3 | E | E | - |
| 65 | S | CH2-C6H5 | H | -CH=CH- | CH3 | Z | E | - |
| 66 | S | CH2-C6H5 | H | CH2-O | CH3 | - | E | - |
| 67 | S | O-C6H5 | H | -CH=CH- | CH3 | E | E | - |
| 68 | O | CH2-C6H5 | H | -CH=CH- | CH3 | E | E | 145 |
| 69 | O | CH2-C6h5 | H | -CH=CH- | CH3 | Z | E | 86 |
| 70 | O | CH2-C6H5 | H | CH2-O | CH3 | - | E | - |
| 71 | S | 2-Cl-C6H4 | H | -CH=CH- | CH3 | E | E | 139 |
| 72 | S | " | H | -CH=CH- | CH3 | Z | E | - |
| 73 | S | " | H | CH2-O | CH3 | - | E | - |
| 74 | S | 3-Cl-C6H4 | H | -CH=CH- | CH3 | Z | E | 130 |
| 75 | S | " | H | CH2-O | CH3 | - | E | 83 |
| 76 | S | 2-OCH3-C6H4 | H | -CH=CH- | CH3 | E | E | - |
| 77 | S | " | H | -CH=CH- | CH3 | Z | E | - |
| 78 | S | 4-Cl-C6H4 | H | -CH=CH- | CH3 | E | E | - |
| 79 | S | " | H | -CH=CH- | CH3 | Z | E | - |
| 80 | S | " | H | CH2-O | CH3 | - | E | - |
| 81 | S | 3-OCH3-C6H4 | H | -CH=CH- | CH3 | E | E | - |
| 82 | S | " | H | -CH=CH- | CH3 | Z | E | - |
| 83 | S | " | H | CH2-O | CH3 | - | E | - |
| 84 | S | 2-F,C6H4 | H | -CH=CH- | CH3 | E | E | 127 |
| 85 | S | " | H | -CH=CH- | CH3 | Z | E | - |
| 86 | S | 3-F,C6H4 | H | -CH=CH- | CH3 | E | E | 156 |
| 87 | S | " | H | -CH=CH- | CH3 | Z | E | - |
| 88 | S | 4-OCH3,C6H4 | H | -CH=CH- | CH3 | E | E | - |
| 89 | S | " | H | -CH=CH- | CH3 | Z | E | - |
| 90 | S | 4-F,C6H4 | H | -CH=CH- | CH3 | E | E | 145 |
| 91 | S | " | H | -CH=CH- | CH3 | Z | E | - |
| 92 | S | 2-6Cl2C6H3 | H | -CH=CH- | CH3 | E | E | 177 |
| 93 | S | " | H | -CH=CH- | CH3 | Z | E | - |
| 94 | S | " | H | CH2-O | CH3 | - | E | - |
| 95 | S | 3,5Cl2,C6H3 | H | -CH=CH- | CH3 | E | E | 158 |
| 96 | S | " | H | -CH=CH- | CH3 | Z | E | 123 |
| 97 | S | " | H | CH2-O | CH3 | - | E | 142 |

TABLEAU IV

(suite)

| EX | X | R1 | R2 | Q | R3 | Q | (2) | F°C |
|---|---|---|---|---|---|---|---|---|
| 98 | S | 2,4Cl2-C6H3 | H | CH2-O | CH3 | - | E | 115 |
| 99 | S | 4Br,3CF3-C6H3 | H | -CH=CH- | CH3 | E | E | 140 |
| 100 | S | " | H | -CH=CH- | CH3 | Z | E | 104 |
| 101 | S | 3-pyridyl | H | -CH=CH- | CH3 | E | E | - |
| 102 | S | " | H | -CH=CH- | CH3 | Z | E | - |
| 103 | S | 3-OCH3,4ClC6H3 | H | CH2-O | CH3 | - | E | 115 |
| 104 | S | 4-pyridyl | H | -CH=CH- | CH3 | E | E | - |
| 105 | S | " | H | -CH=CH- | CH3 | Z | E | - |
| 106 | S | 2-pyridyl | H | -CH=CH- | CH3 | E | E | 140 |
| 107 | S | " | H | -CH=CH- | CH3 | Z | E | - |
| 108 | S | 2CF3-C6H4 | H | -CH=CH- | CH3 | E | E | 143 |
| 109 | S | " | H | -CH=CH- | CH3 | Z | E | - |
| 110 | S | 2-pyrimidinyle | H | -CH=CH- | CH3 | E | E | - |
| 111 | S | " | H | -CH=CH- | CH3 | Z | E | - |
| 112 | S | 3,4-(CH2O2) C6H3 | H | -CH=CH- | CH3 | E | E | 168 |
| 113 | S | " | H | -CH=CH- | CH3 | Z | E | - |
| 114 | S | " | H | CH2-O | CH3 | - | E | 127 |
| 115 | S | 6-pyrimidinyle | H | -CH=CH- | CH3 | E | E | - |
| 116 | S | " | H | -CH=CH- | CH3 | Z | E | - |
| 117 | S | 5-pyrimidinyle | H | -CH=CH- | CH3 | E | E | - |
| 118 | S | " | H | -CH=CH- | CH3 | Z | E | - |
| 119 | S | 2-thiényle | H | -CH=CH- | CH3 | E | E | - |
| 120 | S | " | H | -CH=CH- | CH3 | Z | E | - |
| 121 | S | 2-furyle | H | -CH=CH- | CH3 | E | E | 125 |
| 122 | S | " | H | -CH=CH- | CH3 | Z | E | - |
| 123 | S | 3-furyle | H | -CH=CH- | CH3 | E | E | - |
| 124 | S | " | H | -CH=CH- | CH3 | Z | E | - |
| 125 | S | 4-oxazolyle | H | -CH=CH- | CH3 | E | E | - |
| 126 | S | " | H | -CH=CH- | CH3 | Z | E | - |
| 127 | S | 5-oxazolyle | H | -CH=CH- | CH3 | E | E | - |
| 128 | S | " | H | -CH=CH- | CH3 | Z | E | - |
| 129 | S | 3-CF3-C6H4 | H | -CH=CH- | CH3 | E | E | 142 |
| 130 | S | " | H | -CH=CH- | CH3 | Z | E | - |
| 131 | S | 4-CF3-C6H4 | H | -CH=CH- | CH3 | E | E | 149 |
| 132 | S | " | H | -CH=CH- | CH3 | Z | E | - |
| 133 | S | 2,6F2-C6H3 | H | -CH=CH- | CH3 | E | E | 136 |
| 134 | S | " | H | -CH=CH- | CH3 | Z | E | - |
| 135 | S | 3OCF3-C6H4 | H | -CH=CH- | CH3 | E | E | 125 |
| 136 | S | " | H | -CH=CH- | CH3 | Z | E | - |

TABLEAU IV

(suite)

| EX | X | R1 | R2 | Q | R3 | Q | (2) | F°C |
|---|---|---|---|---|---|---|---|---|
| 137 | S | 4,OCF3-C6H4 | H | -CH=CH- | CH3 | E | E | 122 |
| 138 | S | " | H | -CH=CH- | CH3 | Z | E | - |
| 139 | S | " | H | CH2-O | CH3 | - | E | 92 |
| 140 | S | 3-(C6H5O)-C6H4 | H | -CH=CH- | CH3 | E | E | - |
| 141 | S | " | H | -CH=CH- | CH3 | Z | E | - |
| 142 | S | 4-(C6H5O)-C6H4 | H | -CH=CH- | CH3 | E | E | - |
| 143 | S | " | H | -CH=CH- | CH3 | Z | E | - |
| 144 | S | 3Cl 4,5-(CH2O2) C6H2 | H | -CH=CH- | CH3 | E | E | 144 |
| 145 | S | " | H | -CH=CH- | CH3 | Z | E | 129 |
| 146 | S | 2Cl 4,5-(CH2O2) C6H2 | H | -CH=CH- | CH3 | E | E | 111 |
| 147 | S | " | H | -CH=CH- | CH3 | Z | E | - |
| 148 | S | 3,4-(CH2O2) phénéthyle | H | -CH=CH- | CH3 | E | E | - |
| 149 | S | " | H | -CH=CH- | CH3 | Z | E | - |
| 150 | S | 3,4-(CH2O2) styryle | H | -CH=CH- | CH3 | E | E | 92 |
| 151 | S | " | H | -CH=CH- | CH3 | Z | E | - |
| 152 | S | pipéronyle | H | -CH=CH- | CH3 | E | E | - |
| 153 | S | " | H | -CH=CH- | CH3 | Z | E | - |
| 154 | S | 3,4-Cl2 styryle | H | -CH=CH- | CH3 | E | E | - |
| 155 | S | " | H | -CH=CH- | CH3 | Z | E | - |
| 156 | S | styryle | H | -CH=CH- | CH3 | E | E | 143 |
| 157 | S | " | H | -CH=CH- | CH3 | Z | E | - |
| 158 | S | 4-OCF3-C6H4 | H | CH2-O | CH3 | - | Z | 170 |
| 159 | S | 2-F-C6H4 | H | CH2-O | CH3 | - | E | 105 |
| 160 | S | 3,4(CH2O2)-C6H3 | H | CH2-O | CH3 | - | E | 127 |
| 161 | S | 3,4(CH2O2)-C6H3 | H | CH2-O | CH3 | - | Z | 149 |
| 162 | S | 3,4-(CH2O2)-C6H3-CH2 | H | -CH=CH- | CH3 | E | E | - |
| 163 | S | CH3-CH2- | H | -CH=CH- | CH3 | E | E | 100 |
| 164 | S | CH3-CH2- | H | -CH=CH- | CH3 | Z | E | - |
| 165 | S | 2,3,4,5,6-F-phényl | H | -CH=CH- | CH3 | E | E | 123 |
| 166 | S | 2,3,4,5,6-F-phényl | H | -CH=CH- | CH3 | Z | E | 114 |
| 167 | S | 2,3,4,5-F-phényl | H | -CH=CH- | CH3 | Z | E | 96 |
| 168 | S | 2,3,4,5-F-phényl | H | -CH=CH- | CH3 | E | E | 152 |
| 169 | O | 3,4-Cl2-phényl | H | -CH=CH- | CH3 | E | E | 177 |

(CH2O2) = Méthylènedioxy

<u>Exemple 170</u> : **Préparation d'un concentré émulsifiable**

On a préparé un concentré émulsifiable renfermant comme principe actif, le produit de l'exemple 58.

**ETUDES D'ACTIVITE DES PRODUITS DE L'INVENTION**

**A) METHODES**

I) Activité insecticide :

a) Etude de l'effet sur larves de SPODOPTERA LITTORALIS (S.L.) par contact et ingestion.

On utilise des larves du stade L3 de SPODOPTERA LITTORALIS. On opère à 22°C dans des conditions d'humidité relative de 50 %. On utilise des boîtes de PETRI renfermant un rond de papier filtre humidifié, dans chaque boîte on place deux feuilles de haricot traitées par une solution hydroacétonique (50-50) renfermant le produit à tester.

On fait le comptage des larves mortes au bout de 7 jours. On exprime le résultat en $CL_{50}$ en ppm.

b) Etude de l'effet létal sur Musca Domestica (M.D.) :

On utilise des mouches femelles âgées de 6 jours pesant de 18 à 20 mg.

On opère à 22°C dans des conditions d'humidité relative de 50 %.

Les produits sont administrés par application topique de solution acétonique à 1 g/l sur le thorax dorsal des insectes. Les $CL_{50}$ sont exprimés en ppm.

II) Activité acaricide sur TETRANYCHUS URTICAE (T.U.).

On utilise des plants de haricot comportant 2 feuilles infestées de 30 femelles de Tetranychus Urticae par feuille et mis sous bonette aérée sous plafond lumineux en lumière constante. Les plants sont traités au pistolet Fisher : 4 ml de solution toxique par plant d'un mélange à volume égal d'eau et d'acétone. On laisse sécher pendant une 1/2 heure puis on procède à l'infestation. Les contrôles de mortalité sont effectués au bout de 3 jours.

On recherche la dose $CL_{50}$. Les résultats sont exprimés en ppm.

**B) RESULTATS**

Les résultats sont regroupés dans le tableau ci-dessous, en tenant compte des valeurs suivantes :

A: $CL_{50} \leqq 250$ ppm

B : $250 < CL_{50} < 1000$ ppm

C : $CL_{50} \geq 1000$ ppm

| EXEMPLE | S. L. | M. D. | T. U. |
|---|---|---|---|
| 16 | B | - | - |
| 17 | A | - | B |
| 21 | B | A | B |
| 29 | B | A | A |
| 31 | B | A | A |
| 42 | B | A | - |
| 43 | B | - | - |
| 46 | B | A | B |
| 50 | A | - | A |
| 55 | A | - | A |
| 57 | A | - | A |
| 58 | A | A | A |
| 61 | A | A | - |
| 71 | - | - | A |
| 95 | A | A | - |
| 99 | A | A | A |
| 78 | C | A | C |
| 84 | A | A | A |
| 112 | A | A | A |
| 131 | C | A | A |
| 137 | A | A | A |
| 169 | A | A | A |

## Revendications

**1.** Utilisation des composés de formule générale (I) :

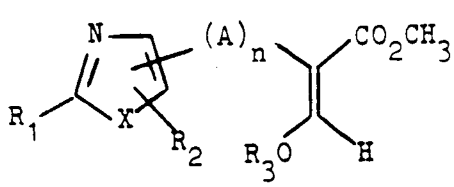

(I)

dans lesquels

- $R_1$ et $R_2$ identiques ou différents représentent un atome d'hydrogène, un atome d'halogène, un radical $CF_3$, un radical hydroxyle, un radical alkyle, alkényle, alkynyle ou thioalkyle renfermant jusqu'à 8 atomes de carbone éventuellement substitué, un radical alkoxy, thioalkoxy ou $SO_2$alkyle renfermant jusqu'à 8 atomes de carbone éventuellement substitué, un radical aryle, aryloxy ou thioaryle renfermant jusqu'à 18 atomes de carbone éventuellement substitué, un radical hétéroaryle ou hétéroaryloxy éventuellement substitué, un radical hétérocyclique à 5 ou 6 chaînons éventuellement substitué ou un radical

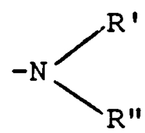

  dans lequel les radicaux R' et R", identiques ou différents représentent un atome d'hydrogène ou un radical alkyle renfermant jusqu'à 8 atomes de carbone ou un radical aryle renfermant jusqu'à 18 atomes de carbone, éventuellement substitué,
- $R_3$ représente un radical alkyle, alkényle ou alkynyle renfermant jusqu'à 8 atomes de carbone éventuellement substitué ou un radical aryle, hétéroaryle renfermant jusqu'à 18 atomes de carbone, un radical hétérocyclique à 5 ou 6 chaînons ou un radical alkoxyalkoxyalkyle renfermant jusqu'à 14 atomes de carbone,
- X représente un atome d'oxygène ou de soufre ou un radical $NR_4$ dans lequel $R_4$ représente un atome d'hydrogène ou un radical alkyle, alkényle ou alkynyle, $SO_2$alkyle, $SO_2$alkényle ou $SO_2$alkynyle renfermant jusqu'à 8 atomes de carbone ou un radical aryle ou $SO_2$aryle renfermant jusqu'à 18 atomes de carbone éventuellement substitué,
- n représente le nombre 0 ou le nombre 1,
- A représente un radical choisi dans le groupe des radicaux suivants :

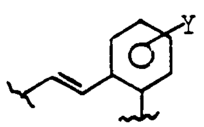 ; 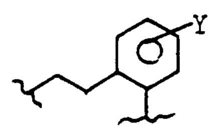 ; 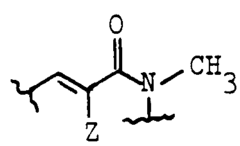 ; 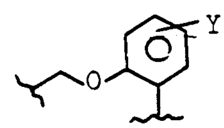

  Y représentant un atome d'hydrogène, un atome d'halogène, un radical $CF_3$, un radical alkyle ou alkoxy renfermant juqu'à 8 atomes de carbone et Z représentant un atome d'hydrogène ou un atome d'halogène,
- la géométrie des doubles liaisons étant E ou Z ou un mélange de géométrie E et Z pour la fabrication de compositions insecticides et/ou acaricides.

**2.** Utilisation selon la revendication 1 des composés de formule (I') :

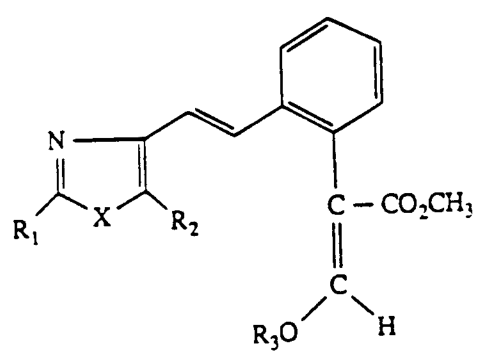

(I')

dans laquelle $R_1$, $R_2$, $R_3$ et X ont la signification indiquée à la revendication 1, la géométrie des doubles liaisons étant E ou Z ou un mélange de géométrie E et Z, pour la fabrication de compositions

insecticides et/ou acaricides.

3. Utilisation selon la revendication 1 ou 2, caractérisé en ce que la géométrie de la double liaison entre l'hétérocycle et le noyau aromatique est (E).

4. Utilisation selon l'une quelconque des revendications 1 à 3, caractérisé en ce que la géométrie de la double liaison du groupement alkoxy acrylate est (E).

5. Utilisation selon l'une quelconque des revendications 1 à 4, caractérisé en ce que $R_2$ représente un atome d'hydrogène.

6. Utilisation selon l'une quelconque des revendications 1 à 5, caractérisée en ce que X représente un atome de soufre.

7. Utilisation selon l'une quelconque des revendications 1 à 5, caractérisée en ce que X représente un atome d'oxygène.

8. Utilisation selon l'une quelconque des revendications 1 à 7, caractérisée en ce que $R_1$ représente un radical alkyle renfermant jusqu'à 3 atomes de carbone éventuellement substitué.

9. Utilisation selon la revendication 8, caractérisée en ce que $R_1$ représente un radical éthyle, isopropyle ou trifluorométhyle.

10. Utilisation selon l'une quelconque des revendications 1 à 7, caractérisée en ce que $R_1$ représente un radical phényle non substitué ou substitué par un ou plusieurs atomes d'halogène.

11. Utilisation selon la revendication 10, caractérisée en ce que l'halogène est le chlore, le brome ou le fluor.

12. Utilisation selon l'une quelconque des revendications 1 à 7, caractérisée en ce que $R_1$ représente un radical phényle substitué par un radical trifluorométhyle ou trifluorométhoxy.

13. Utilisation selon l'une quelconque des revendications 1 à 7, caractérisée en ce que $R_1$ représente un radical phényle substitué par un radical méthylènedioxy.

14. Utilisation selon l'une quelconque des revendications 1 à 7, caractérisée en ce que $R_1$ représente un radical benzyle.

15. Utilisation selon l'une quelconque des revendications 1 à 4, caractérisée en ce que le composé de formule (I) est choisi parmi les composés dont les noms suivent :
   - alpha-[(E)-(méthoxyméthylène)] 2-[(E)-2-[2-(2-éthyl 4-thiazolyl) 4-thiazolyl] éthényl] benzèneacétate de méthyle,
   - alpha-[(E)-(méthoxyméthylène)] 2-[(E)-2-[2-(3,4-dichloro phényl) 4-thiazolyl] éthényl] benzèneacétate de méthyle,
   - alpha-[(E)-(méthoxyméthylène)] 2-[(E)-2-(2-trifluorométhyl) 4-thiazolyl) éthényl] benzèneacétate de méthyle,
   - alpha-[(E)-(méthoxyméthylène)] 2-[(E)-2-[2-(4-chlorophényl) 4-thiazolyl) éthényl] benzèneacétate de méthyle,
   - alpha-[(E)-(méthoxyméthylène)] 2-[(E)-2-[2-(2-fluorophényl) 4-thiazolyl) éthényl] benzèneacétate de méthyle,
   - alpha-[(E)-(méthoxyméthylène)] 2-[(E)-2-[2-(3-trifluorométhyl4-bromophényl) 4-thiazolyléthényl] benzèneacétate de méthyle,
   - alpha-[(E)-(méthoxyméthylène)] 2-[(E)-2-[2-(1,3-benzodioxol-5-yl) 4-thiazolyl) éthényl] benzèneacétate de méthyle,
   - alpha-[(E)-(méthoxyméthylène)] 2-[(E)-2-[2-(4-trifluorométhylphényl) thiazolyl) éthényl] benzèneacétate de méthyle,
   - alpha-[(E)-(méthoxyméthylène)] 2-[(E)-2-[2-(4-trifluorométhoxyphényl) 4-thiazolyl) éthényl] benzèneacétate de méthyle,

- alpha-[(E)-(méthoxyméthylène)] 2-[(E)-2-[2-(3,4-dichloro phényl) 4-oxazolyl] éthényl] benzèneacétate de méthyle.

**16.** A titre de produits chimiques nouveaux, les produits de formule (I) telle que définie à la revendication 1, répondant à la formule (I') telle que définie à la revendication 2 dont les noms suivent :
   - alpha-[(E)-(méthoxyméthylène)] 2-[(E)-2-[2-(2-éthyl 4-thiazolyl) 4-thiazolyl] éthényl] benzèneacétate de méthyle,
   - alpha-[(E)-(méthoxyméthylène)] 2-[(E)-2-[2-(3,4-dichloro phényl) 4-thiazolyl] éthényl] benzèneacétate de méthyle,
   - alpha-[(E)-(méthoxyméthylène)] 2-[(E)-2-(2-trifluorométhyl) 4-thiazolyl) éthényl] benzèneacétate de méthyle,
   - alpha-[(E)-(méthoxyméthylène)] 2-[(E)-2-[2-(4-chlorophényl) 4-thiazolyl) éthényl] benzèneacétate de méthyle,
   - alpha-[(E)-(méthoxyméthylène)] 2-[(E)-2-[2-(2-fluorophényl) 4-thiazolyl) éthényl] benzèneacétate de méthyle,
   - alpha-[(E)-(méthoxyméthylène)] 2-[(E)-2-[2-(3-trifluorométhyl4-bromophényl) 4-thiazolyléthényl] benzèneacétate de méthyle,
   - alpha-[(E)-(méthoxyméthylène)] 2-[(E)-2-[2-(1,3-benzodioxol-5-yl) 4-thiazolyl) éthényl] benzèneacétate de méthyle,
   - alpha-[(E)-(méthoxyméthylène)] 2-[(E)-2-[2-(4-trifluorométhylphényl) thiazolyl) éthényl] benzèneacétate de méthyle,
   - alpha-[(E)-(méthoxyméthylène)] 2-[(E)-2-[2-(4-trifluorométhoxyphényl) 4-thiazolyl) éthényl] benzèneacétate de méthyle,
   - alpha-[(E)-(méthoxyméthylène)] 2-[(E)-2-[2-(3,4-dichloro phényl) 4-oxazolyl] éthényl] benzèneacétate de méthyle.

**17.** Les composés de formule (I) telle que définie à la revendication 1, caractérisés en ce que $R_1$ et $R_2$ identiques ou différents représentent un radical hydroxyle ou un radical

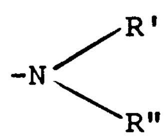

dans lequel R′ et R″ identiques ou différents représentent un atome d'hydrogène ou un radical alkyle renfermant jusqu'à 8 atomes de carbone ou un radical aryle renfermant jusqu'à 18 atomes de carbone, éventuellement substitué.

**18.** Les composés de formule (I′) telle que définie à la revendication 2, caractérisés en ce que $R_1$ et $R_2$ identiques ou différents représentent un radical hydroxyle ou un radical

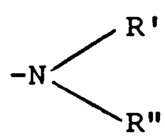

dans lequel R′ et R″ identiques ou différents représentent un atome d'hydrogène ou un radical alkyle renfermant jusqu'à 8 atomes de carbone ou un radical aryle renfermant jusqu'à 18 atomes de carbone, éventuellement substitué.

## Claims

1. Use of the compounds of general formula (I):

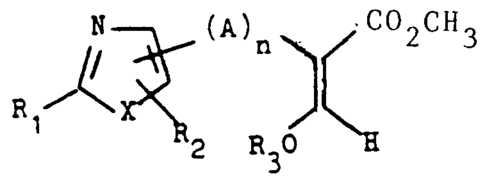

(I)

in which

- $R_1$ and $R_2$, identical or different, represent a hydrogen atom, a halogen atom, a $CF_3$ radical, a hydroxyl radical, an optionally substituted alkyl, alkenyl, alkynyl or thioalkyl radical containing up to 8 carbon atoms, an optionally substituted alkoxy, thioalkoxy or $SO_2$alkyl radical containing up to 8 carbon atoms, an optionally substituted aryl, aryloxy or thioaryl radical containing up to 18 carbon atoms, an optionally substituted heteroaryl or heteroaryloxy radical, an optionally substituted heterocyclic radical with 5 or 6 members or an

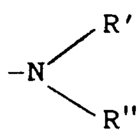

radical in which the R' and R'' radicals, identical or different, represent a hydrogen atom or an alkyl radical containing up to 8 carbon atoms or an aryl radical containing up to 18 carbon atoms, optionally substituted,
- $R_3$ represents an optionally substituted alkyl, alkenyl or alkynyl radical containing up to 8 carbon atoms or an aryl, heteroaryl radical containing up to 18 carbon atoms, a heterocyclic radical with 5 or 6 members or an alkoxyalkoxyalkyl radical containing up to 14 carbon atoms,
- X represents an oxygen or sulphur atom or an $NR_4$ radical in which $R_4$ represents a hydrogen atom or an alkyl, alkenyl or alkynyl, $SO_2$alkyl, $SO_2$alkenyl or $SO_2$alkynyl radical containing up to 8 carbon atoms or an optionally substituted aryl or $SO_2$aryl radical containing up to 18 carbon atoms,
- n represents the number 0 or the number 1,
- A represents a radical chosen from the following group of radicals:

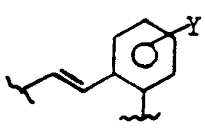

;

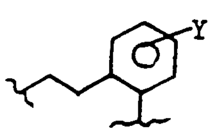

;

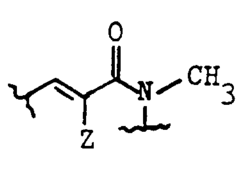

;

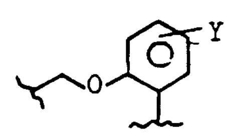

Y representing a hydrogen atom, a halogen atom, a $CF_3$ radical, an alkyl or alkoxy radical containing up to 8 carbon atoms and Z representing a hydrogen atom or a halogen atom,
- the geometry of the double bonds being E or Z or a mixture of E and Z geometry, for the preparation of insecticide and/or acaricide compositions.

**2.** Use according to claim 1 of the compounds of formula (I'):

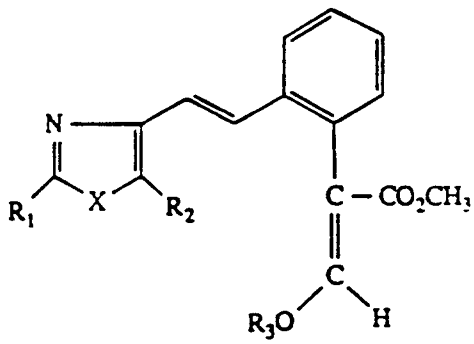

(I')

in which $R_1$, $R_2$, $R_3$ and X have the meaning indicated in claim 1, the geometry of the double bonds being E or Z or a mixture of E and Z geometry, for the preparation of insecticide and/or acaricide compositions.

**3.** Use according to claim 1 or 2, characterized in that the geometry of the double bond between the heterocycle and the aromatic nucleus is (E).

**4.** Use according to any one of claims 1 to 3, characterized in that the geometry of the double bond of the alkoxy acrylate group is (E).

**5.** Use according to any one of claims 1 to 4, characterized in that $R_2$ represents a hydrogen atom.

**6.** Use according to any one of claims 1 to 5, characterized in that X represents a sulphur atom.

**7.** Use according to any one of claims 1 to 5, characterized in that X represents an oxygen atom.

**8.** Use according to any one of claims 1 to 7, characterized in that $R_1$ represents an optionally substituted alkyl radical containing up to 3 carbon atoms.

**9.** Use according to claim 8, characterized in that $R_1$ represents an ethyl, isopropyl or trifluoromethyl radical.

**10.** Use according to any one of claims 1 to 7, characterized in that $R_1$ represents a phenyl radical non-substituted or substituted by one or more halogen atoms.

**11.** Use according to claim 10, characterized in that the halogen is chlorine, bromine or fluorine.

**12.** Use according to any one of claims 1 to 7, characterized in that $R_1$ represents a phenyl radical substituted by a trifluoromethyl or trifluoromethoxy radical.

**13.** Use according to any one of claims 1 to 7, characterized in that $R_1$ represents a phenyl radical substituted by a methylenedioxy radical.

**14.** Use according to any one of claims 1 to 7, characterized in that $R_1$ represents a benzyl radical.

**15.** Use according to any one of claims 1 to 4, characterized in that the compound of formula (I) is chosen from the compounds the names of which follow:

- methyl alpha-[(E)-(methoxymethylene)] 2-[(E)-2-[2-(2-ethyl 4-thiazolyl) 4-thiazolyl] ethenyl] benzeneacetate,
- methyl alpha-[(E)-(methoxymethylene)] 2-[(E)-2-[2-(3,4-dichloro phenyl) 4-thiazolyl] ethenyl] benzeneacetate,
- methyl alpha-[(E)-(methoxymethylene)] 2-[(E)-2-(2-trifluoromethyl) 4-thiazolyl) ethenyl] benzeneacetate,

- methyl alpha-[(E)-(methoxymethylene)] 2-[(E)-2-[2-(4-chlorophenyl) 4-thiazolyl) ethenyl] benzeneacetate,
- methyl alpha-[(E)-(methoxymethylene)] 2-[(E)-2-[2-(2-fluorophenyl) 4-thiazolyl) ethenyl] benzeneacetate,
- methyl alpha-[(E)-(methoxymethylene)] 2-[(E)-2-[2-(3-trifluoromethyl 4-bromophenyl) 4-thiazolylethenyl] benzeneacetate,
- methyl alpha-[(E)-(methoxymethylene)] 2-[(E)-2-[2-(1,3-benzodioxol-5-yl) 4-thiazolyl) ethenyl] benzeneacetate,
- methyl alpha-[(E)-(methoxymethylene)] 2-[(E)-2-[2-(4-trifluoromethylphenyl) thiazolyl) ethenyl] benzeneacetate,
- methyl alpha-[(E)-(methoxymethylene)] 2-[(E)-2-[2-(4-trifluoromethoxyphenyl) 4-thiazolyl) ethenyl] benzeneacetate,
- methyl alpha-[(E)-(methoxymethylene)] 2-[(E)-2-[2-(3,4-dichloro phenyl) 4-oxazolyl] ethenyl] benzeneacetate.

**16.** As new chemical products, the products of formula (I) as defined in claim 1, corresponding to formula (I') as defined in claim 2 of which the names follow:
- methyl alpha-[(E)-(methoxymethylene)] 2-[(E)-2-[2-(2-ethyl 4-thiazolyl) 4-thiazolyl] ethenyl] benzeneacetate,
- methyl alpha-[(E)-(methoxymethylene)] 2-[(E)-2-[2-(3,4-dichloro phenyl) 4-thiazolyl] ethenyl] benzeneacetate,
- methyl alpha-[(E)-(methoxymethylene)] 2-[(E)-2-(2-trifluoromethyl) 4-thiazolyl) ethenyl] benzeneacetate,
- methyl alpha-[(E)-(methoxymethylene)] 2-[(E)-2-[2-(4-chlorophenyl) 4-thiazolyl) ethenyl] benzeneacetate,
- methyl alpha-[(E)-(methoxymethylene)] 2-[(E)-2-[2-(2-fluorophenyl) 4-thiazolyl) ethenyl] benzeneacetate,
- methyl alpha-[(E)-(methoxymethylene)] 2-[(E)-2-[2-(3-trifluoromethyl 4-bromophenyl) 4-thiazolylethenyl] benzeneacetate,
- methyl alpha-[(E)-(methoxymethylene)] 2-[(E)-2-[2-(1,3-benzodioxol-5-yl) 4-thiazolyl) ethenyl] benzeneacetate,
- methyl alpha-[(E)-(methoxymethylene)] 2-[(E)-2-[2-(4-trifluoromethylphenyl) thiazolyl) ethenyl] benzeneacetate,
- methyl alpha-[(E)-(methoxymethylene)] 2-[(E)-2-[2-(4-trifluoromethoxyphenyl) 4-thiazolyl) ethenyl] benzeneacetate,
- methyl alpha-[(E)-(methoxymethylene)] 2-[(E)-2-[2-(3,4-dichloro phenyl) 4-oxazolyl] ethenyl] benzeneacetate.

**17.** The compounds of formula (I) as defined in claim 1, characterized in that $R_1$ and $R_2$, identical or different, represent a hydroxyl radical or an

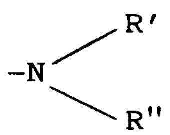

radical in which R' and R'', identical or different, represent a hydrogen atom or an alkyl radical containing up to 8 carbon atoms or an aryl radical containing up to 18 carbon atoms, optionally substituted.

**18.** The compounds of formula (I') as defined in claim 2, characterized in that $R_1$ and $R_2$, identical or different, represent a hydroxyl radical or an

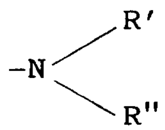

radical in which R' and R", identical or different, represent a hydrogen atom or an alkyl radical containing up to 8 carbon atoms or an aryl radical containing up to 18 carbon atoms, optionally substituted.

**Patentansprüche**

1. Verwendung der Verbindungen der allgemeinen Formel (I):

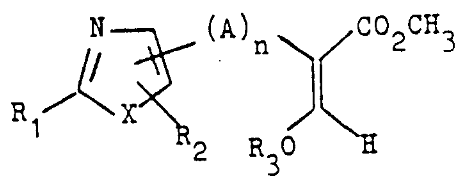

(I)

in denen

- $R_1$ und $R_2$, identisch oder verschieden, ein Wasserstoffatom, ein Halogenatom, einen $CF_3$-Rest, einen Hydroxylrest, einen gegebenenfalls substituierten Alkyl-, Alkenyl-, Alkinyl- oder Thioalkylrest, der bis zu 8 Kohlenstoffatome umfaßt, einen gegebenenfalls substituierten Alkoxy-, Thioalkoxy- oder $SO_2$-Alkylrest, der bis zu 8 Kohlenstoffatome umfaßt, einen gegebenenfalls substituierten Aryl-, Aryloxy- oder Thioarylrest, der bis zu 18 Kohlenstoffatome umfaßt, einen gegebenenfalls substituierten Heteroaryl- oder Heteroaryloxyrest, einen gegebenenfalls substituierten heterocyclischen Rest mit 5 oder 6 Gliedern oder einen Rest

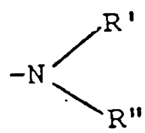

darstellen, in dem die Reste R' und R", identisch oder verschieden, ein Wasserstoffatom oder einen bis zu 8 Kohlenstoffatome umfassenden Alkylrest oder einen bis zu 18 Kohlenstoffatome umfassenden Arylrest, der gegebenenfalls substituiert ist, darstellen,
- $R_3$ einen gegebenenfalls substituierten Alkyl-, Alkenyl- oder Alkinylrest, der bis zu 8 Kohlenstoffatome umfaßt, oder einen Aryl-, Heteroarylrest, der bis zu 18 Kohlenstoffatome umfaßt, einen heterocyclischen Rest mit 5 oder 6 Gliedern oder einen Alkoxyalkoxyalkylrest, der bis zu 14 Kohlenstoffatome umfaßt, darstellt,
- X ein Sauerstoff- oder Schwefelatom oder einen Rest $NR_4$ darstellt, in dem $R_4$ ein Wasserstoffatom oder einen bis zu 8 Kohlenstoffatome umfassenden Alkyl-, Alkenyl- oder Alkinyl-, $SO_2$-Alkyl-, $SO_2$-Alkenyl- oder $SO_2$-Alkinylrest oder einen bis zu 18 Kohlenstoffatome umfassenden Aryl- oder $SO_2$-Arylrest, der gegebenenfalls substituiert ist, darstellt,
- n die Zahl 0 oder die Zahl 1 darstellt,
- A einen Rest darstellt, der aus der Gruppe der folgenden Reste ausgewählt ist:

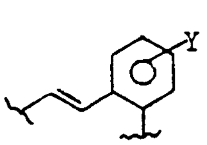 ; 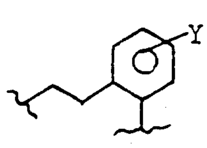 ; 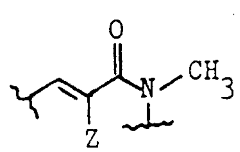 ; 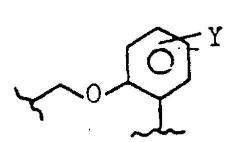

wobei Y ein Wasserstoffatom, ein Halogenatom, einen $CF_3$-Rest, einen bis zu 8 Kohlenstoffatome umfassenden Alkyl- oder Alkoxyrest darstellt und Z ein Wasserstoffatom oder ein Halogenatom darstellt,

- wobei die Geometrie der Doppelbindungen E oder Z oder eine Mischung der Geometrie E und Z ist,

für die Herstellung von Insektizid- und/oder Akarizid-Zusammensetzungen.

**2.** Verwendung nach Anspruch 1 der Verbindungen der Formel (I'):

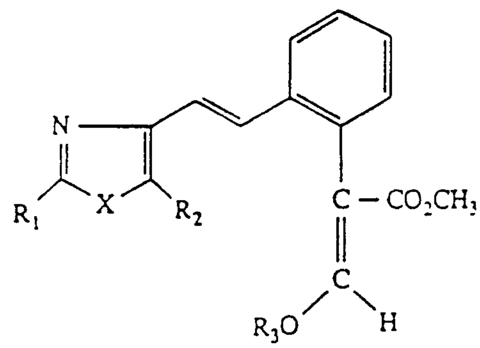

in der $R_1$, $R_2$, $R_3$ und X die in Anspruch 1 angegebene Bedeutung aufweisen, wobei die Geometrie der Doppelbindungen E oder Z oder einer Mischung der Geometrie E und Z ist, für die Herstellung von Insektizid- und/oder Akarizid-Zusammensetzungen.

**3.** Verwendung nach Anspruch 1 oder 2, dadurch gekennzeichnet, daß die Geometrie der Doppelbindung zwischen dem Heterocyclus und dem aromatischen Kern (E) ist.

**4.** Verwendung nach irgendeinem der Ansprüche 1 bis 3, dadurch gekennzeichnet, daß die Geometrie der Doppelbindung der Alkoxyacrylat-Gruppe (E) ist.

**5.** Verwendung nach irgendeinem der Ansprüche 1 bis 4, dadurch gekennzeichnet, daß $R_2$ ein Wasserstoffatom darstellt.

**6.** Verwendung nach irgendeinem der Ansprüche 1 bis 5, dadurch gekennzeichnet, daß X ein Schwefelatom darstellt.

**7.** Verwendung nach irgendeinem der Ansprüche 1 bis 5, dadurch gekennzeichnet, daß X ein Sauerstoffatom darstellt.

**8.** Verwendung nach irgendeinem der Ansprüche 1 bis 7, dadurch gekennzeichnet, daß $R_1$ einen gegebenenfalls substituierten Alkylrest darstellt, der bis zu 3 Kohlenstoffatome umfaßt.

**9.** Verwendung nach Anspruch 8, dadurch gekennzeichnet, daß $R_1$ einen Ethyl-, Isopropyl- oder Trifluormethylrest darstellt.

**10.** Verwendung nach irgendeinem der Ansprüche 1 bis 7, dadurch gekennzeichnet, daß $R_1$ einen unsubstituierten oder durch ein oder mehrere Halogenatome substituierten Phenylrest darstellt.

**11.** Verwendung nach Anspruch 10, dadurch gekennzeichnet, daß das Halogen Chlor, Brom oder Fluor ist.

**12.** Verwendung nach irgendeinem der Ansprüche 1 bis 7, dadurch gekennzeichnet, daß $R_1$ einen durch einen Trifluormethyl- oder Trifluormethoxyrest substituierten Phenylrest darstellt.

**13.** Verwendung nach irgendeinem der Ansprüche 1 bis 7, dadurch gekennzeichnet, daß $R_1$ einen durch einen Methylendioxyrest substituierten Phenylrest darstellt.

**14.** Verwendung nach irgendeinem der Ansprüche 1 bis 7, dadurch gekennzeichnet, daß $R_1$ einen Benzylrest darstellt.

**15.** Verwendung nach irgendeinem der Ansprüche 1 bis 4, dadurch gekennzeichnet, daß die Verbindung der Formel (I) ausgewählt ist aus den Verbindungen, deren Namen folgen:

- Methyl-alpha-[(E)-(methoxymethylen)]-2-[(E)-2-[2-(2-ethyl-4-thiazolyl)-4-thiazolyl]ethenyl]-phenylacetat,
- Methyl-alpha-[(E)-(methoxymethylen)]-2-[(E)-2-[2-(3,4-dichlorphenyl)-4-thiazolyl]ethenyl]-phenylacetat,
- Methyl-alpha-[(E)-(methoxymethylen)]-2-[(E)-[2-(2-trifluormethyl)-4-thiazolyl]ethenyl]phenylacetat,
- Methyl-alpha-[(E)-(methoxymethylen)]-2-[(E)-2-[2-(4-chlorphenyl)-4-thiazolyl]ethenyl]phenylacetat,
- Methyl-alpha-[(E)-(methoxymethylen)]-2-[(E)-2-[2-(2-fluorphenyl)-4-thiazolyl]ethenyl]phenylacetat,
- Methyl-alpha-[(E)-(methoxymethylen)]-2-[(E)-2-[2-(3-trifluormethyl-4-bromphenyl)-4-thiazolyl]-ethenyl]phenylacetat,
- Methyl-alpha-[(E)-(methoxymethylen)]-2-[(E)-2-[2-(1,3-benzodioxo-5-yl)-4-thiazolyl]ethenyl]-phenylacetat,
- Methyl-alpha-[(E)-(methoxymethylen)]-2-[(E)-2-[2-(4-trifluormethylphenyl)thiazolyl]ethenyl]-phenylacetat,
- Methyl-alpha-[(E)-(methoxymethylen)]-2-[(E)-2-[2-(4-trifluormethoxyphenyl)-4-thiazolyl]ethenyl]-phenylacetat,
- Methyl-alpha-[(E)-(methoxymethylen)]-2-[(E)-2-[2-(3,4-dichlorphenyl)-4-oxazolyl]ethenyl]-phenylacetat.

**16.** Als neue chemische Produkte die Produkte der Formel (I), wie in Anspruch 1 definiert, die der Formel (I'), wie in Anspruch 2 definiert, entsprechen, deren Namen folgen:

- Methyl-alpha-[(E)-(methoxymethylen)]-2-[(E)-2-[2-(2-ethyl-4-thiazolyl)-4-thiazolyl]ethenyl]-phenylacetat,
- Methyl-alpha-[(E)-(methoxymethylen)]-2-[(E)-2-[2-(3,4-dichlorphenyl)-4-thiazolyl]ethenyl]-phenylacetat,
- Methyl-alpha-[(E)-(methoxymethylen)]-2-[(E)-2-[2-(trifluormethyl)-4-thiazolyl]ethenyl]phenylacetat,
- Methyl-alpha-[(E)-(methoxymethylen)]-2-[(E)-2-[2-(4-chlorphenyl)-4-thiazolyl]ethenyl]phenylacetat,
- Methyl-alpha-[(E)-(methoxymethylen)]-2-[(E)-2-[2-(2-fluorphenyl)-4-thiazolyl]ethenyl]phenylacetat,
- Methyl-alpha-[(E)-(methoxymethylen)]-2-[(E)-2-[2-(3-trifluormethyl-4-bromphenyl)-4-thiazolyl]-ethenyl]phenylacetat,
- Methyl-alpha-[(E)-(methoxymethylen)]-2-[(E)-2-[2-(1,3-benzodioxo-5-yl)-4-thiazolyl]ethenyl]-phenylacetat,
- Methyl-alpha-[(E)-(methoxymethylen)]-2-[(E)-2-[2-(4-trifluormethylphenyl)thiazolyl]ethenyl]-phenylacetat,
- Methyl-alpha-[(E)-(methoxymethylen)]-2-[(E)-2-[2-(4-trifluormethoxyphenyl)-4-thiazolyl]ethenyl]-phenylacetat,
- Methyl-alpha-[(E)-(methoxymethylen)]-2-[(E)-2-[2-(3,4-dichlorphenyl)-4-oxazolyl]ethenyl]-phenylacetat.

**17.** Verbindungen der Formel (I), wie in Anspruch 1 definiert, dadurch gekennzeichnet, daß $R_1$ und $R_2$, identisch oder verschieden, einen Hydroxylrest oder einen Rest

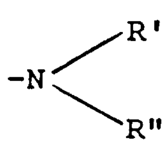

darstellen, in dem R' und R'', identisch oder verschieden, ein Wasserstoffatom oder einen bis zu 8 Kohlenstoffatome umfassenden Alkylrest oder einen bis zu 18 Kohlenstoffatome umfassenden Arylrest, der gegebenenfalls substituiert ist, darstellen.

**18.** Verbindungen der Formel (I'), wie in Anspruch 2 definiert, dadurch gekennzeichnet, daß $R_1$ und $R_2$, identisch oder verschieden, einen Hydroxylrest oder einen Rest

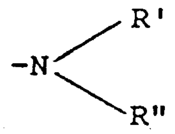

darstellen, in dem R' und R'', identisch oder verschieden, ein Wasserstoffatom oder einen bis zu 8 Kohlenstoffatome umfassenden Alkylrest oder einen bis zu 18 Kohlenstoffatome umfassenden Arylrest, der gegebenenfalls substituiert ist, darstellen.